# EUROPEAN PATENT SPECIFICATION

(11) **EP 0 482 988 B1**
(45) Date of publication and mention of the grant of the patent: **17.01.1996**
(21) Application number: 91402793.3
(22) Date of filing: 21.10.1991
(51) Int. Cl.: A61H 1/00

(54) **Therapeutic device for correcting misalignment of center of gravity of human body**
Therapeutische Vorrichtung zum Korrigieren der falschen Ausrichtung des Schwerpunktes eines menschlichen Körpers
Dispositif thérapeutique pour corriger le mauvais alignement du centre de gravité du corps humain

(30) Priority: 22.10.1990 JP 285151/90; 20.12.1990 JP 404512/90; 01.08.1991 JP 193253/91
(43) Date of publication of application: 29.04.1992
(73) Proprietor: Fukunaga, Mikio, Yokohama-shi, Kanagawa-ken (JP)
(72) Inventor: Fukunaga, Mikio, Yokohama-shi, Kanagawa-ken (JP)
(74) Representative: Dubois-Chabert, Guy

(56) References cited:
- DE-C- 658 848
- FR-A- 897 782
- FR-A- 1 532 069
- FR-A- 2 328 487
- FR-A- 2 462 157
- US-A- 4 951 654

## Description

### Field of the Invention

This invention concerns a therapeutic device for correcting an abnormal curvature or a human spine which has been deformed by scoliosis or another malfunction, and restoring the correct center of gravity of the human body when the center of gravity has been misaligned by same.

### Description of the Prior Art

Conventionally, traction was used as a means of correcting an improper curvature of the human spine, which forms the main element of the human skeleton, caused by scoliosis or another malfunction.

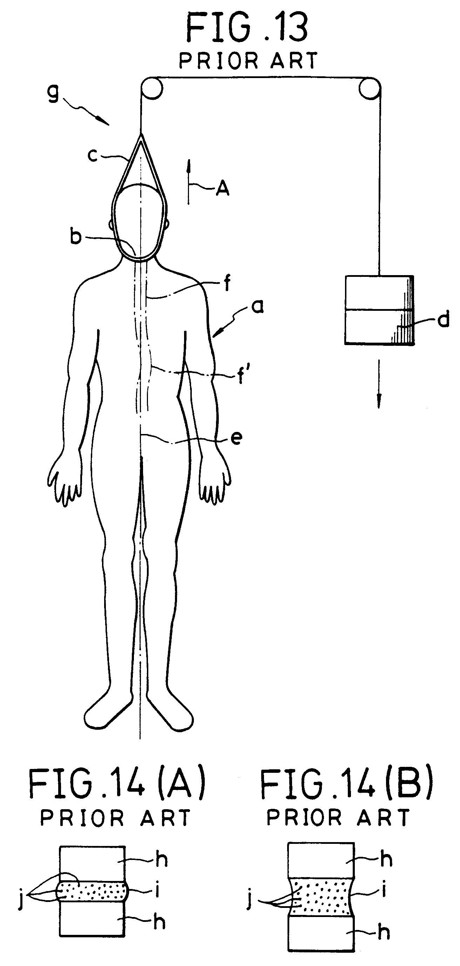

As shown in Fig. 13 one such traction device g comprises a rope c which is attached to the chin b of the body a, and the chin b is pulled in the direction of elongation of the spine e (direction A) of the body a by a weight d or a spring, not shown, so as to bring a crooked part f' of the spinal portion f into line with the axial line of the body a.

However, as shown in Figs. 14(A) and (B), the spine f comprises vertebrae h and pieces of cartilage i which are interposed between the vertebrae. If, therefore, traction therapy is applied using the aforesaid spinal curvature correction device g, the pieces of cartilage i are pulled out of their normal state as shown in Fig. 14(A) so that they become elongated as shown in Fig. 14(B).

If the pieces of cartilage i are elongated in this way, the density of nutrients j in the pieces of cartilage i changes from fine to coarse. As a result, the nutrients are absorbed with difficulty by the vertebrae h. This may cause the inherent strength of the spine f to be weakened in its role as the main element of the human skeleton.

### Summary of the Invention

The authors of the invention conceived the idea that if the cartilage in the human spine were compressed by applying a compression to the spine from the head towards the body, nutrients could be absorbed easily by the vertebrae so that the spine as a whole would be strengthened, and in addition the natural healing mechanism of the human body would be stimulated so that the spine would return to a normal linear state.

It is therefore an object of the invention to provide a device for correcting the center of gravity of the human body which can be used for applying such a compression therapy, and which can also be used for correcting spinal curvature or misalignment of the center of gravity of the human body, without any adverse effect on the vertebrae.

To achieve the above objective, the therapeutic device of this invention for correcting the center of gravity of the human body comprises a head contact member placed in contact with the head, and head compression means for pushing the head contact member towards the head.

Further, another therapeutic device according to the invention comprises a head contact member which comes into contact with the head, head compression means which pushes the member towards the head, and body compression means which push body contact members placed in contact with the body towards the body.

Unlike prior apparatus from the prior art such as FR-A-2 328 487, where a solid mass presses on the head, the head contact member is filled with a gaseous, liquid or soft the patient's head.

Attention must also be paid to US-A64,951,654 the subject of which is an orthopedic table with a sling looped on the forehead and tensioned to impact flexion and compression to the neck and a cushion under the loins to curve the spine there.

### Brief Description of the Attached Drawings

FIG. 1 is a rear view of a device for correcting the center of gravity of the human body illustrating a first embodiment of the invention.

FIG. 2 is a view of the base of the contact surface of the head contact member according to the invention.

FIG. 3(A) is an enlarged view illustrating the state of the spine before compression is applied.

FIG. 3(B) is an enlarged view illustrating the state of the spine during compression.

FIG.4 is a sectional view of a device for correcting the center of gravity of the human body illustrating a second embodiment of the invention.

FIG.5 is a sectional view of a device for correcting the center of gravity of the human body illustrating a third embodiment of the invention.

FIG.6 is a lateral view of a device for correcting the center of gravity of the human body illustrating a fourth embodiment of the invention.

FIG.7 is a lateral view of a device for correcting the center of gravity of the human body illustrating an fifth embodiment of the invention.

FIG.8 is a front view of a device for correcting the center of gravity of the human body illustrating an sixth embodiment of the invention.

FIG.9 is a lateral view of a device for correcting the center of gravity of the human body illustrating a sixth embodiment of the present invention.

FIG.10 is a plan view illustrating a device for correcting the center of gravity of the human body.

FIG.11 is a front view of a device for correcting the center of gravity of the human body illustrating a seventh embodiment of the invention.

FIG.12 is a lateral view of a device for correcting the center of gravity of the human body illustrating a seventh embodiment of the invention.

FIG.13 is a front view showing the way in which a conventional device for correcting spinal curvature was used.

FIG.14A is an enlarged view showing the state of the spine before using a conventional device for correcting spinal curvature.

FIG.14B is an enlarged view showing the state of the spine after using a conventional device for correcting spinal curvature.

### Description of the Preferred Embodiments

The therapeutic device for correcting the center of gravity of the human body according to the invention will now be described with reference to the drawings.

### (Embodiment 1)

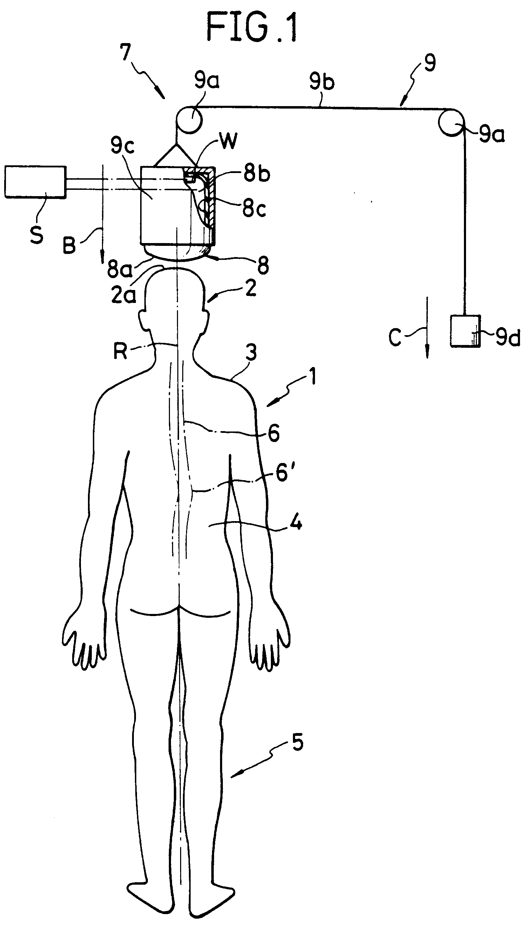

FIG. 1 - FIG. 3 illustrate the first embodiment of the invention.

In FIG. 1, 1 is the body of a patient being treated, 2 is the head, 3 are the shoulders, 4 is the back including the waist, 5 is a leg, and 6 is the spine. 7 is a head compression device for correcting the center of gravity of the human body, and comprises a head contact member 8 which comes into contact with the head 2 of the body 1, and a pressure regulator 9 which regulates the compression applied by this head contact member 8 to the body 1.

The pressure regulator 8 comprises a support 9c attached to one end of rope 9b suspended between pulleys 9a and 9b, and a pressure regulating member 9d attached to the other end of the rope 9b. The support 9c is shaped like a box which is open on its underside, and it contains the head contact member 8.

The pressure regulating member 9d comprises a plurality of weights whose combined force acts in the opposite direction (FIG. 1, direction C) to that of the compression applied by head contact member 8 (FIG. 1, direction B), and which can be individually attached or detached so as regulate the compression applied by the head contact member 8.

Alternatively, a plurality of pressure regulating members 9d of different weight may be provided, and a suitable one attached according to the compression it is desired to apply.

The head contact member has a one-piece construction in the form of a closed bag. It comprises a contact surface 8a in contact with the vertex 2a of the head 2, and its interior 8c is filled with a compression substance 8b which acts as a head compression means.

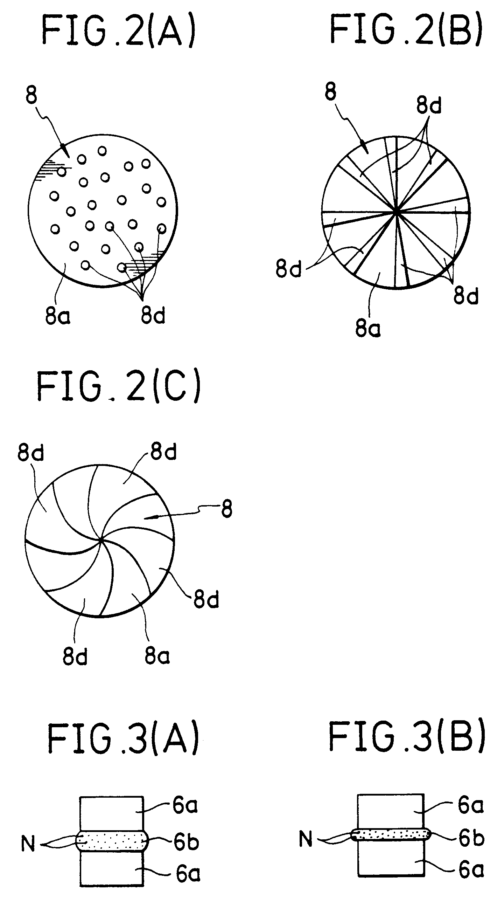

The contact surface 8a protrudes from the support 9c of the pressure regulator 9, and is formed of an elastic, pliable film with sealtight properties that can adapt its shape to that of the vertex 2a of the head 2. This film may consist for example of an elastic material such as rubber or the like, cloth or paper coated with rubber, vinyl chloride or polyethylene. Further, the contact surface 8a may have a plurality of projections 8d formed in a one-piece construction, as shown in FIG. 2(A) - 2(C), such that when the surface is applying a compression to the head 2, friction between the surface 8a and the head 2 is prevented and the head 2 is massaged at the same time.

The part of the head contact member 8 contained in the support 9c and the contact surface 8a protruding from the support 9c may also be formed of different materials.

The compressing substance 8b must be of such a type that when the contact surface 8a comes into contact with the vertex 2a of the head 2, the deformed shape of the surface 8a is maintained and a uniform surface pressure is applied to the head 2. Examples of substances that can be used are water, mercury or other liquids, powders such as sand or iron sand, and gels such as gelatin.

Further, an oscillator W generating ultrasonic waves or the like may be installed in the interior 8c of the head contact member 8 so that the vibrations of the oscillator are transmitted to the head 2 and massage it.

Further, a controller S may be provided in the head compression device 7 to vary the compression applied by the compressing substance to the head 2 from 1 - 160 times per minute. Further, this controller S may also be connected to the oscillator W to control the frequency of oscillation.

In the aforesaid construction, the body 1 is first positioned such that its center axis R is in a straight line, and the contact surface 8a of the head contact member 8 is brought into contact with the vertex 2a of the head 2.

The interior 8c of the head contact member 8 is then filled with a compressing substance 8b (mercury is the most suitable on account of its high specific gravity) so that the weight of this substance is 10 - 50% of the weight of the body 1.

Further, if the weight of the compressing substance 8b is uncomfortable depending on the age of the patient, a suitable pressure (or weight) regulating member 9d is attached to the rope 9b so as adjust the compression (weight) applied to the head 2.

When a surface pressure set at a suitable value is applied to the head 2, the cartilage 6b between the vertebrae 6a, 6a which before compression is in the state shown in FIG. 3(A), is compressed as shown in FIG. 3(B) under the action of the compression applied to the head 2.

Nutrients N in the cartilage 6b, which before compression had the density shown in FIG. 3(A), therefore become much denser as shown in FIG. 3(B), so that they are easily absorbed by the vertebrae 6a, 6a.

Further, by repeatedly applying a compression such that the normal state shown in FIG. 3(A) alternates with the state shown in FIG. 3(B), there is a pumping effect on the spine which causes the nutrients N in the cartilage 6b to be even more easily absorbed by the vertebrae 6a.

### (Embodiment 2)

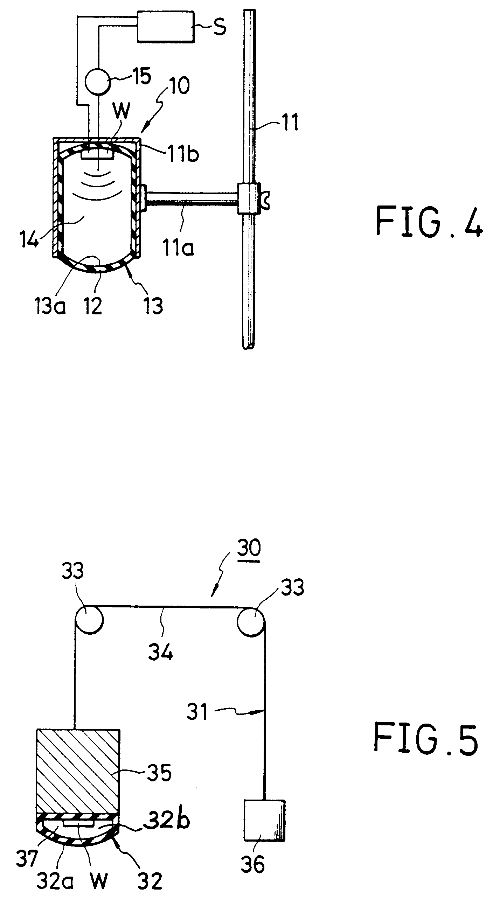

FIG. 4 illustrates a second embodiment of the invention. According to this embodiment, a head compression device 10 which acts as a device for correcting the center of gravity of the human body, is attached to a pipe 11 via a bracket 11a such that it can be moved up and down or fixed on the pipe.

Further, a head contact member 13 having a contact surface 12 is installed in a box 11b fixed at one end of the bracket 11a.

The interior 13a of this head contact member 13 is filled with a substance 14 which may be a gas such as air, or a liquid such as water or mercury.

The compression may be adjusted by varying the amount of substance 14 filling the interior 13a of the head contact member 13 via a pump 15 which acts as a head compression means.

Further, an oscillator W may be installed in the head contact member 13, the oscillator W and the pump 15 both being connected to a controller S in order to control the oscillator vibration frequency of the oscillator W and the compression variation frequency of the pump 15.

### (Embodiment 3)

FIG. 5 illustrates a third embodiment of the invention. According to this embodiment, a head compression device 30 for correcting the center of gravity of the human body comprises a pressure regulator 31 and a contact surface 32a.

The pressure regulator 31 comprises a weight 35 attached to one end of a rope 34 suspended between springs 33, 33, and a pressure regulating member 36 attached to the other end of the rope 34.

The weight 35 may consist of a heavy, hard object such as a metal or concrete block.

The pressure regulating member 36 acts in the opposite direction to the compression direction of the weight 35. A plurality of pressure regulating members 36 may be individually attached or detached so an to regulate the compression (weight) of the weight 35.

Alternatively, a plurality of pressure regulating members 36 of different weight may be provided, and a suitable one attached according to the compression it is desired to apply.

The head contact member 32 is fixed to the lower end of the weight 35, and consists of a member of one-piece construction such as a bag which can change its shape (for example of rubber or cloth) having a contact surface 32a.

The interior 32b of the head contact member 32 is filled with a substance 37 of such a type that when the member comes into contact with the head of the patient, the deformed shape of the member is maintained and a uniform surface pressure is applied to the head. Examples of shape-maintaining substances are liquids such as water or mercury, powders such as sand or iron sand, gels such as gelatin, gases such as air, waste thread or cotton.

The substance 37 is chosen to suit the material of the bead contact member 32. For example, if the substance 37 is mercury, the member may be of rubber, and if the substance 37 is cotton, the member may be of cloth (or rubber).

### (Embodiment 4)

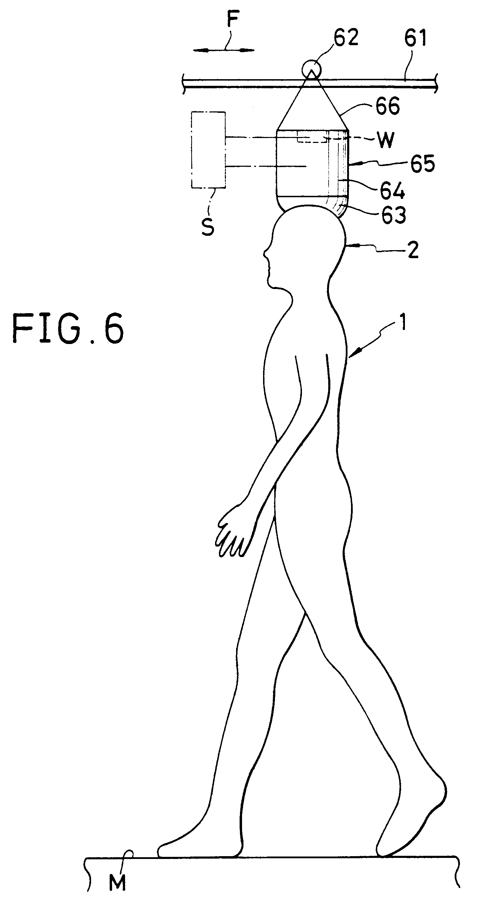

FIG. 6 illustrates a fourth embodiment of the invention. According to this embodiment, the head 2 of the body 1 is compressed while the patient remains free to walk.

In this embodiment, a rail 61 is provided which is horizontal with respect to the floor M, and a pulley 62 is provided on this rail such that it is free to slide.

A head compression device 65 for correcting the center of gravity of a human body having a head contact member 63 and head compression means 64 is provided with a rope 66. By attaching this rope 66 to the pulley 62, the head compression device 65 is rendered able to move on the rail (in direction F shown in FIG. 6 ).

Further, a plurality of ropes of different lengths are provided to suit patients of different height.

Further, an oscillator W may be installed in the head contact member 63, the oscillator W and the head compression means 64 both being connected to a controller S in order to control the oscillator vibration frequency of the oscillator W and the compression variation frequency of the head compression means 64. The head Compression means 64 used in this embodiment is the same as in the first and second embodiments mentioned above.

### (Embodiment 5)

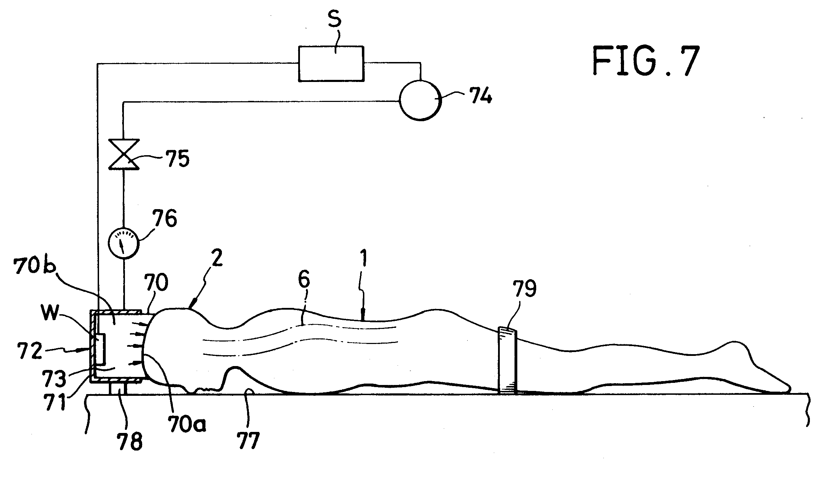

FIG. 7 illustrates a fifth embodiment of the invention. According to this embodiment, the head 2 of the patient can be compressed with the patient supine (either lying on his back or side).

A head compression device 72 for correcting the center of gravity of the human body comprises a head contact member 70 having a contact surface 70a and a protective cover 71. The interior 70b of the head contact member 70 is filled with a substance 73 which may be a liquid such as water or mercury, or a gas such as air, and the compression applied by the head contact member 70 is adjusted by varying the amount of filling substance 73 via a pump 74 which acts as a head compression means. In the figure, 75 is a valve, 76 is a pressure meter, 77 is a floor surface with which the body is in contact, 78 is a fixing member for the purpose of fixing the protective cover 71 to the floor surface 77, and 79 is a body restraining strap.

Further, an oscillator W may be installed in the head contact member 70, the oscillator W and the pump 74 both being connected to a controller S in order to control the oscillator vibration frequency of the oscillator W and the compression variation frequency of the pump 74.

This apparatus can also perform human-handed treatment such as pushing out an abnormally curved protruding part of the spine 6 while compressing the head 2 of the body 1.

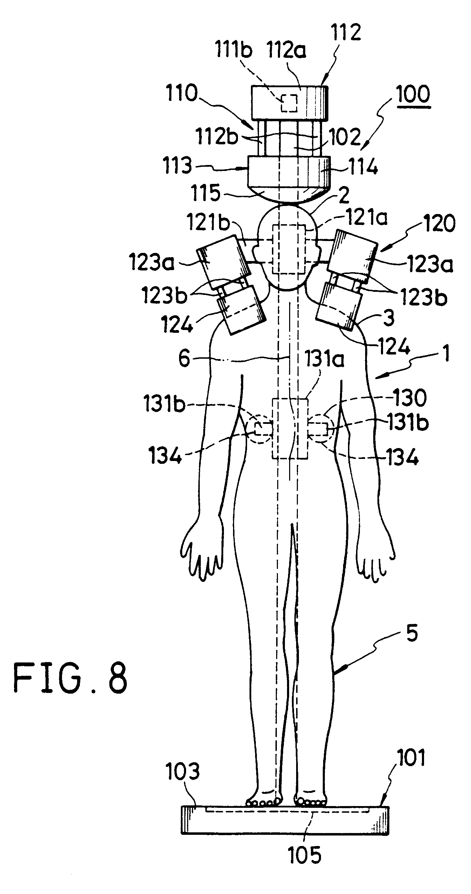

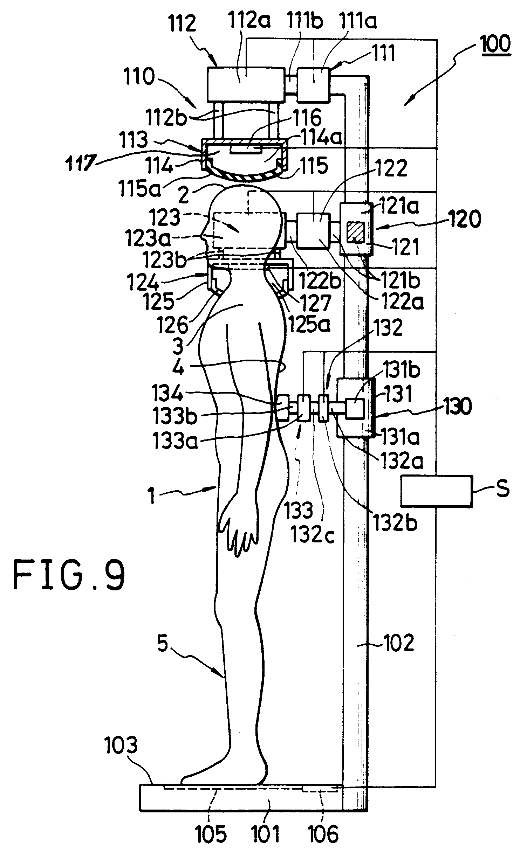

FIGS. 8-10 show a sixth embodiment of this invention. In this embodiment, a device 100 for correcting the center of gravity of the human body comprises a head compression device 110, a shoulder compression device 120 and a back compression device 130 for applying compression respectively to the head 2, shoulders 3 and back 4 of a patient receiving compression therapy.

The device 100 for correcting the center of gravity of the human body comprises a platform 101 and a pillar 102 mounted on one side of the platform 101.

An outline of human feet 104 is printed on the upper surface 103 of the platform 101 to indicate the position in which the patient should stand. The platform 101 also has a built-in detecting device 105 (e.g. a sheet pressure sensor) for detecting the center of gravity of the body 1, and an output means 106 which outputs information from the center of gravity detecting means 105 to a controller S.

The head compression device 110 comprises a horizontal cylindrical member 111 and a head compression means 112. The horizontal cylindrical member 111 used here further comprises a cylinder 111a attached to the upper extremity of the pillar 102 and a rod 111b, the arrangement being such that the head compression means 112 can be displaced between the head 2 of the body 1 and the pillar 102 by the elongation or contraction of the rod 111b. Further, the horizontal cylindrical member 111 is arranged such that it can be rotated or fixed in a vertical plane.

The head compression means 112 comprises a cylinder 112a attached to the rods 111b of the horizontal cylindrical member 111, a plurality of rods 112b, 112b... attached to the cylinder 112a, and a head contact member 113 attached to the ends of the rods 112b, 112b... Further, the rode 112b, 112b... are arranged such that they can elongate and contract independently in a vertical direction.

The head contact member 113 comprises a box 114 open underneath and a bead contact material 115 consisting of an elastic, pliant material such as rubber or the like. The interior 114a of the box 114 is made sealtight by the head contact material 115. Further the interior 114a of the box 114 is filled with substance 117 (a gas such as air or a liquid such as water or mercury). Further, an oscillator 116 generating ultrasonic waves or the like is installed in the interior 114a of the box 114.

The horizontal cylindrical member 111, head contact means 112 and oscillator 116 are electrically connected to the controller S. The arrangement is such that the contact direction and the applied pressure of the head contact means 112, or the vibration frequency of the oscillator, can be controlled based on center of gravity position data from the center of gravity detection device 105 built in the platform 101 and on previously set values (e.g. age, sex, etc.).

The shoulder compression device 120 comprises a bracket 121 positioned above the pillar 102, a pair of horizontal cylindrical members 122, 122 attached to the left and right of the bracket 121, and shoulder compression means 123, 123 attached to the horizontal cylindrical members 122, 122.

The bracket 121 comprises a fixing member 121a supported by the pillar 102, and supporting members 121b, 121b which project to the left and right of this fixing member 121a. Further, the bracket 121 is attached such that it can either slide longitudinally or be fixed on the pillar 102, in order that its height can be adjusted to the position of the shoulders 3 of the body 1.

The horizontal cylindrical members 122 each comprise a cylinder 122a and a rod 122b attached to the pillar 102 which are arranged such that they can elongate or contract in a horizontal direction between the shoulders 3, 3 of the body 1 and the pillar 102. Further, the horizontal cylindrical members 122 are arranged such that they can be rotated or fixed in a vertical plane.

The shoulder compression means 123 each comprise cylinders 123a attached to the rods 122b of the horizontal cylindrical members 122, a plurality of rods 123b, 123b... attached to these cylinders 123a, and shoulder contact pieces 124 attached to the ends of the rods 123b, 123b... Further, the rods 123b, 123b... are arranged such that they can independently elongate or contract in a vertical plane.

The shoulder contact pieces 124 each comprise a box 125 open underneath and a head contact material 126 consisting of an elastic, pliant material such as rubber or the like. The interior 125a of the box 125 is made sealtight by the head contact material 126. Further, the interior 125a of the box 125 is filled with a gas such as air, or a liquid such as water or mercury. Further, an oscillator 127 generating ultrasonic waves or the like is installed in the interior of the box 125.

The horizontal cylindrical members 122, shoulder compression means 123 and oscillator 127 are electrically connected to the controller S. The arrangement is such that the contact direction and the applied pressure of the shoulder compression means 123, or the vibration frequency of the oscillator, can be controlled based on center of gravity position data from a center of gravity detection device and on previously set values (e.g. age, sex, etc).

The back compression device 130 comprises a pair of curved pieces 132, 132 attached to a bracket 131 located in the middle of the pillar 102, and back compression means 133 attached to the curved pieces 132, 132.

The bracket 131 comprises a fixing piece 131a supported by the pillar 102, and supporting pieces 131b, 131b which project to the left and right of this fixing piece 131a. Further, the bracket 131 is attached such that it can either slide longitudinally or be fixed on the pillar 102 acting as a rail in order that its height can be adjusted to the height, etc., of the body 1.

The curved pieces 132 each comprise a fixing member 132a attached to the pillar 102, a curved member 132b attached to the fixing member 132a, and a supporting member 132c attached to the curved member 132b.

The back compression means 133 each comprise a cylinder 133a connected to a supporting member 132c which is attached to a curved member 132b, a rod 133b attached to the cylinder 133a, and a contact piece 134 attached to the end of the rod 133b.

The curved pieces 132 and back compression means 133 are lectrically connected to a controller S, and the arrangement is such that the contact direction and the applied pressure of the back contact means 133 can be controlled based on center of gravity position data from a center of gravity detection device and on previously set values (e.g. age, sex, etc.).

In this arrangement, the patient is first made to stand on the platform 101. The contact pieces 113, 124 and 134 of the head compression device 110, the shoulder compression device 120 and the back compression device 130 are then brought respectively into contact with the head 2, the shoulders 3 and the back 4 of the body 1.

Next, the head compression device 110 is made to apply a compression to the extent of 3% - 50% of the weight of the patient's body (approximately 75% in the case of sportsmen and other muscular people). The direction in which the compression is applied by the head compression device 110 to the body 1 may be suitably chosen depending on the patient's condition (e.g. the direction in which the spine 6 is curved).

When a compression is applied by the head compression device 101 to the head 2 of the body 1, a compression may also be applied simultaneously to the shoulders 3 or the back 4 of the body 1 by the shoulder compression device 120 or the back compression device 130.

In such a case, if a compression is applied to the shoulders 3 by the shoulder compression device 120 at the same time as a compression is applied to the head 2 by the head compression device 110, the combined compression of the two devices is arranged to be 3% - 75% of the weight of the body 1, and is arranged such that the compression applied to the head 2 is 50% - 90% of the combined compression. Further, if a compression is applied to the back 4 by the back compression device 130 at the same time as a compression is applied to the head 2 by the head compression device 110, the back compression device 130 is adjusted such that it compresses the curved part of the spine 6 of the body 1 toward the center line of the body 1, thereby ensuring that the compression exerted by the head compression device 110 acts efficiently on the spine 6.

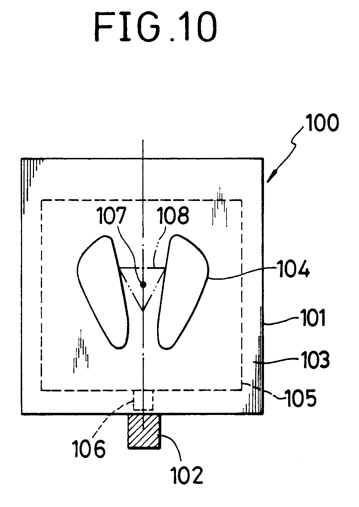

Further, the apparatus can be so arranged that no control of the direction and magnitude of the compression is performed if the center of gravity of the body 1 detected by the center of gravity detecting means 105 lies within an ideal area 108 defined by the periphery of the center of gravity 107 of a healthy person, as shown in FIG. 10.

Further, the compressions applied by the compression means 112, 123 and 133 can either be applied continuously for a suitable period or varied within predetermined limits, and in addition, can either be applied in a fixed direction for a suitable period or applied while varying their direction.

The case has been described wherein a compression is applied while the patient is standing, but the platform 101 of the device 100 for correcting the center of gravity of the human body may be arranged in the form of a seat (with the patient sitting, kneeling or cross-legged).

### (Embodiment 7)

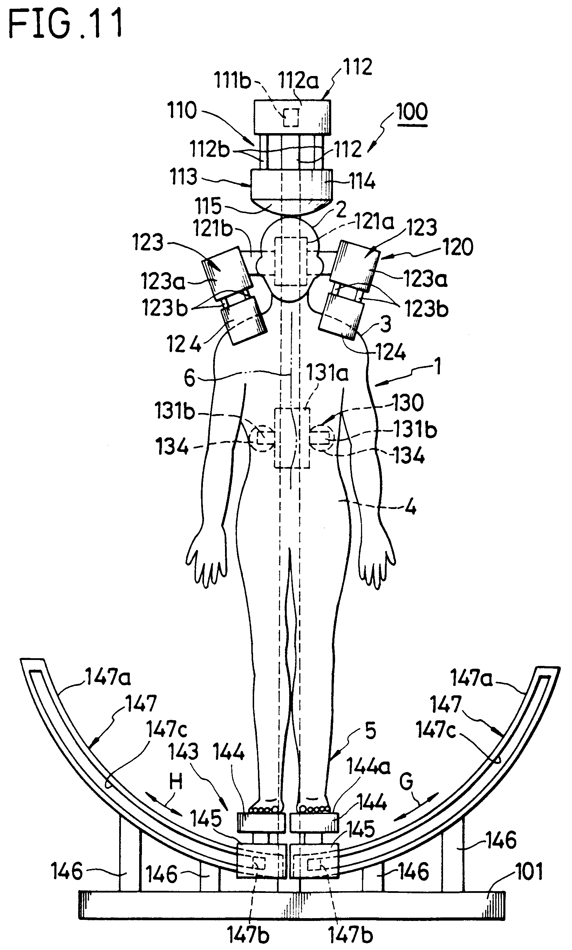

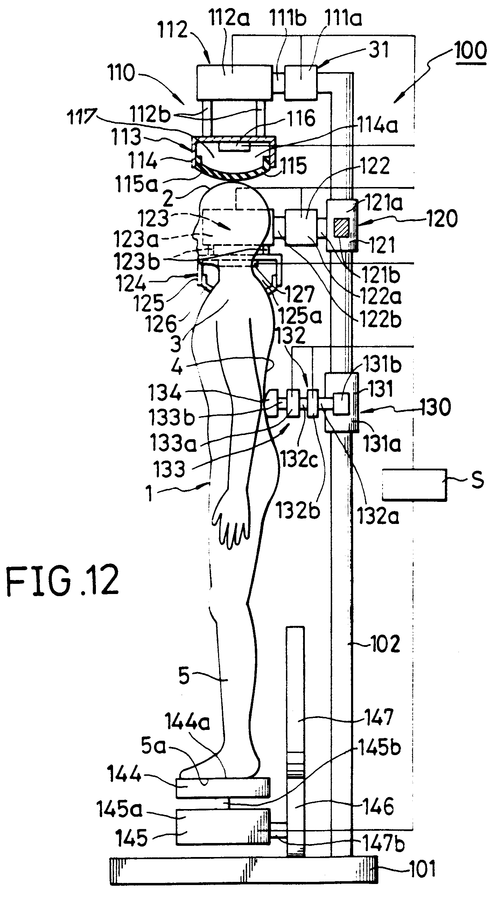

FIG. 11 and FIG. 12 illustrate a seventh embodiment of the invention. According to this embodiment, the apparatus for correcting the center of gravity of the human body is provided with a device for applying a compression to the feet of the body 1 of a patient receiving compression therapy.

The device 100 for correcting the center of gravity of the human body comprises a platform 101, and a pillar 102 mounted on one side of the platform 101. To this pillar 102 are attached a head compression device 10, a shoulder compression device 120 and a back compression device 130 for applying compressions respectively to the head 2, shoulders 3 and back 4 of the body 1 of the patient. Further, a foot compression device 143 is disposed on the platform 101 such that a compression can be applied from the feet 5 towards the body 1.

The foot compression device 143 comprises a pair of left and right foot compression members 144, 144 in contact with the soles 5a, 5a of the left and right feet of the body 1, and foot compression means 145, 145 for compressing the pair of left and right foot compression members 144, 144 towards the body. Further, the foot compression device 143 is attached to moving and fixing mechanisms 147, 147 to separate or bring together the feet of the patient via supporting members 146, 146 fixed to the platform 101.

The foot compression members 144 are each attached to a rod 145b of a compression means 145 consisting of a cylinder 145a and a rod 145b so that a predetermined compression can be applied to the body 1 via the elongation and contraction of the rods 145b. Further, the foot contact members 144 are arranged such that the compression applied to the body 1 is controlled by a controller S electrically connected to the foot compression means 145. Further, the magnitude and direction of the compression (e.g. an oblique compression in the forwards, backwards or left and right) applied by the foot compression means 145 can be set independently for the left and right sides. Further, a plurality of projections, not shown, protrudes from the contact surfaces 144a of the foot contact members 144 in order to stimulate the soles of the feet 5a.

The moving and fixing mechanism 147 comprises an arc-shaped rail 147a which separates or brings together the feet of the body 1, and supporting pieces 147b which connect this rail 147a and the foot compression means 145. A groove 147c is also formed on the rail 147a such that the foot compression means 145 can slide (directions G and H in FIG. 11) due to the sliding and fixing action of the supporting pieces 147b along the groove 147c.

In this arrangement, the patient is first made to stand on the contact surfaces 144a, 144a of the pair of left and right foot contact members 144, 144, and the contact pieces 113, 124 and 134 of the head compression device 110, the shoulder compression device 130 and the back compression device 130 are brought into contact with the head 2, shoulders 3 and back 4 of the body 1.

Next, the head compression device 110 is made to apply a compression to the extent of 3% - 50% of the weight of the patient's body (approximately 75% in the case of sportsmen and other muscular people). The direction in which the compression is applied by the head compression device 110 to the body 1 may be suitably chosen depending on the patient's condition (e.g. the direction in which the spine 6 is curved).

Further, when a compression is applied by the head compression device 110 to the head 2 of the body 1, a compression may also be applied simultaneously to the feet 5, the shoulders 3 or the back 4 of the body 1 by the foot compression device 143, the shoulder compression device 120 or the back compression device 130.

In such a case, if an upward compression is applied to the feet 5 by the foot compression device 143 at the same time as a compression is applied to the head 2 by the head compression device 110, the combined compression of the two devices is arranged to be 3% - 75% the weight of the body 1, and is arranged such that the compression applied to the head 2 is 50% - 90% of the combined compression. Further, if the center of gravity of the body 1 is incorrect, the foot compression device 143 is displaced via the motion and fixing mechanism 147 so as to separate the feet, and a compression is applied by the foot compression means 145 on one side which is greater than the compression applied by the foot compression means 145 on the other side so as to correct the position if the center of gravity.

Further, if a compression is applied to the shoulders 3 by the shoulder compression device 120 at the same time as a compression is applied to the head 2 by the head compression device 110, the combined compression of the two devices is arranged to be 3% - 75% of the weight of the body 1, and is arranged such that the compression applied to the head 2 is 50% - 90% of the combined compression.

Further, if a compression is applied to the back 4 by the back compression device 130 at the same time as a compression is applied to the head 2 by the head compression device 110, the back compression device 130 is adjusted such that it compresses the curved part of the spine 6 of the body 1 toward the center axis of the body 1, thereby ensuring that the compression exerted by the head compression device 110 acts efficiently on the spine 6.

Further, the compressions applied by the compression means 145, 112 and 133 can either be applied continuously for a suitable period or varied within predetermined limits.

Further, the moving and fixing mechanism 147 can also be arranged such that the feet 5 can be separated in the front-back direction of the body 1, and the foot contact members 144 can also be displaced in a complex direction between back-front and left-right.

Further, in the aforesaid embodiment, the device for correcting the center of gravity of the human body is used with the patient standing, but it may also be arranged such that the patient is in a sitting or lying posture.

It is to be understood that instead of the head compression device 110 disclosed in the sixth and seventh embodiments, any of the head compression devices 7, 10, 30, 40, 80 (body contact member), disclosed in the aforesaid embodiments 1-3 and in the embodiment 5, can be used. Further, the head compression devices 7, 10, 30, 40, 80 (body contact member), disclosed in the aforesaid embodiments 1 -3 end in the embodiment 5, may also be used in conjunction with the shoulder compression device 120, back compression device 130 and foot compression device 143 disclosed in the sixth and seventh embodiments.

As described hereintofore, this invention comprises a head compression member which comes into contact with the patient's head, and a head compression means which compresses the aforesaid head contact member towards the patient's head. It can therefore be used as a device for correcting the center of gravity which can be applied to compression therapy without any adverse effect on the vertebrae.

Further, as the invention comprises a head contact member which comes into contact with the head, a head compression means which compresses the aforesaid contact member towards the head, and body compression means which compress body contact members with which the body is in contact towards the patient's body, not only are spinal deformities corrected more efficiently, but also displacements of the center of gravity due to factors other than spinal deformity are corrected efficiently.

Further, if the head contact member is used with the patient in a standing posture, a compression may be applied to the body in a straight line.

Further, if the head contact member is used with the patient in a sitting posture, a compression may be applied essentially from the head to waist of the body.

Further, if the head contact member is used with the patient in a lying posture, a compression may be applied with the muscles of the body relaxed. The distance between where the muscles start and stop working is then short, and the compression is applied with the whole body in a relaxed condition.

Further, if the head contact member is used with the patient walking, a compression may be applied under conditions which the patient meets in ordinary day-to-day life.

Further, as the contact surface of the head contact member consists of an elastic, pliant film, a compression may be applied all over the head and there is no waste of pressure. Further, the same effect may be obtained if the contact surface of the head contact member has a shape which fits that of the patient's head.

Further, if projections are formed on the contact surface of the head contact member which comes into contact with the patient, the scalp can be massaged at the same time as a compression is applied to the head, and the contact surface is prevented from slipping when the compression is applied.

Further, as the aforesaid compression means have a mechanical construction, a carefully controlled compression can be applied to the patient.

Further, if the aforesaid compression means are human means applied by the patient or another person, the apparatus for correcting the center of gravity is simple to operate and economical.

Moreover, if the aforesaid compression means is a gravitational means depending on the weight of an object, a constant compression can be applied uniformly.

Further, as the compression means are provided with means for varying direction with respect to the body, the center of gravity of the body may be corrected from different directions.

Further, if the head contact member and the shoulder contact member which comes into contact with the shoulders is formed in a one-piece construction, the head shoulders may be compressed by one compression means.

Further, as one of the body compression means is a shoulder compression device having shoulder contact members which come into contact with the shoulders and shoulder compression means which compress these contact members towards the body, the compression is distributed all over the body without being concentrated only on the head.

Further, as another of the body compression means is a back compression device having back contact members which come into contact with the back and back compression means which compress these contact members towards the body, a spinal deformity can be pushed towards the center at the same time as a compression is applied from the head, and the apparatus for correcting the center of gravity is thereby rendered even more effective.

Further, as another of the body compression means is a foot compression device having foot contact members which come into contact with the soles of the feet and foot compression means which compress these contact members towards the body, the body can be compressed in the up and down direction.

Moreover, by combining the shoulder compression device, back compression device and foot compression device, a more complex correction of the center of gravity can be performed.

Further, by arranging that the contact direction of the contact members can be varied according to center of gravity position information from the center of gravity detecting device, spinal deformities can be observed and corrected automatically with a fine degree of control, and displacements of the center of gravity due to factors other than spinal deformity can also be corrected efficiently.

Further, by separating or bringing together the patient's feet by means of the moving and fixing mechanism of the foot compression means, the direction in which a compression is applied from beneath the patient can be set as desired, and displacements of the center of gravity may be corrected.

## Claims

1. An apparatus for correcting the center of gravity of a human person who has at least a body (1), a head (2) and a spinal column (6), comprising:
a head contact member (8, 13, 32, 63, 70, 115) which is placed in contact with the person's head (2), and
compression means (9, 14, 15, S, 33, 34, 35, 36, 64, 73, 74, 111a, 111b, 112a, 112b, 113, 114, 114a, 115) for biasing said head contact member against the head (2) so as to compress at least a portion of the spinal column (6).
CHARACTERIZED IN THAT said head contact member (8, 13, 32, 63, 70, 115) includes an interior (8c, 13a, 32b, 70b, 114a) which is filled with a fluid substance (8b; 14; 37; 73; 117) in order to apply an essentially uniform surface pressure to the person's head (2), the interior (8c, 13a, 32b, 70b, 114a) and the fluid substance (8b, 14, 37, 73, 117) forming part of means which applies an essentially uniform surface pressure to the person's head (2).

2. An apparatus as set forth in claim 1, CHARACTERIZED IN THAT said compression means further includes a counter weight (9d) which is connected to said head contact member by a rope (9b), the counter weight being variable to selectively reduce the amount of pressure applied by said head contact member (8, 13, 32, 63, 70, 115) by the weight of the fluid substance in said interior.

3. An apparatus as set forth in any of the preceding claims CHARACTERIZED IN THAT said head contact member (8, 13, 32, 41, 70, 115) further includes an oscillator (W) which is arranged to produce vibrations which are transmitted to the person's head by way of the fluid substance (air, water, mercury, sand, iron fillings, gelatin gel, waste cotton etc.,) in said interior.

4. An apparatus as set forth in any of the preceding claims CHARACTERIZED BY a pump (74) and a valve (75) which controls the amount of pressure in said interior and therefore the pressure applied to the head of the patient alone or in combination with the generation of vibration by said oscillator.

5. An apparatus as set forth in and of the preceding claims CHARACTERIZED IN THAT said compression means is mounted on a rail (61) parallel to the floor (M) by way of a pulley (62) so that the compression means can move along below the rail (61) and the person may walk along under said rail (61) while said head contact member applies a spinal compressing force.

6. An apparatus as set forth in any of the preceding claims CHARACTERIZED IN THAT said compression means is adapted to apply a force of between 3% and 75% of the person's body weight.

7. An apparatus as set forth in any of the preceding claims CHARACTERIZED BY means (120) for applying pressure to the patient's body while the spine of the patient is compressed in a manner which corrects the configuration of the patient's spine while said spine is compressed.

8. An apparatus as set forth in claim 7, CHARACTERIZED IN THAT said means (120, 130) for applying pressure to the patient's spine apply independent compressions to correct the configuration of the center of gravity position of the patient based on the center of gravity position data from the patient.

9. An apparatus set forth in claim 7, CHARACTERIZED IN THAT said body pressure applying means comprises left and right shoulder compression means (120, 123a, 123b, 124) which can apply different compressions to the left and right shoulders of the person, respectively, each of said left and right shoulder compression means having an oscillator (127) included therein.

10. An apparatus as set forth in any of claims 7 and 9, CHARACTERIZED IN THAT the combined compression applied by both said compression means and said body pressure applying means is in the range of 50% - 90% of the person's body weight.

11. An apparatus set forth in any of claims 7, 9, and 10, CHARACTERIZED IN THAT said body pressure applying means comprises a pair of arcuate guide tracks (147a, 147a) on which left and right foot compression members (144, 144) are respectively movably mounted, each of the left end right foot compression members being individually adjustable so as to enable different forces to be applied to the person's feet.

## Patentansprüche

1. Apparat zum Korrigieren des Schwerpunktes einer menschlichen Person, die zumindest einen Körper (1), einen Kopf (2) und eine Wirbelsäule (6) hat, welcher aufweist:
ein Kopfkontaktglied (8,13,32,63,70,115), das in Kontakt mit dem Kopf (2) der Person gebracht ist, und
Druckmittel
(9,14,15,S,33,34,35,36,64,73,74,111a,111b,112a, 112b,113,114,114a,115) zum Drücken des Kopfkontaktgliedes gegen den Kopf (2), um wenigstens einen Teil der Wirbelsäule (6) zusammenzudrücken,
**dadurch gekennzeichnet,**
daß das Kopfkontaktglied (8,13,32,63,70,115) ein Inneres (8c,13a,32b,70b,114a) enthält, das mit einer fluiden Substanz (8b;14;37;73;117) gefüllt ist, um einen im wesentlichen gleichförmigen Oberflächendruck auf den Kopf (2) der Person auszuüben, wobei das Innere (8c,13a,32b,70b,114a) und die fluide Substanz
(8b,14,37,73,117) einen Teil von Mitteln bilden, die einen im wesentlichen gleichförmigen Oberflächendruck auf den Kopf (2) der Person ausüben.

2. Apparat nach Anspruch 1, dadurch gekennzeichnet, daß die Druckmittel weiterhin ein Gegengewicht (9d) aufweisen, das mit dem Kopfkontaktglied durch ein Seil (9b) verbunden ist, wobei das Gegengewicht veränderlich ist zum wahlweisen Herabsetzen der Größe des von dem Kopfkontaktglied (8,13,32,63,70,115) durch das Gewicht der fluiden Substanz im Inneren ausgeübten Druckes.

3. Apparat nach einem der vorhergehenden Ansprüche, dadurch gekennzeichnet, daß das Kopfkontaktglied (8,13,32,41,70,115) weiterhin einen Oszillator (W) enthält, der so angeordnet ist, daß er Vibrationen erzeugt, die mittels der fluiden Substanz (Luft, Wasser, Quecksilber, Sand, Eisenfüllung, Gelatine-Gel, Baumwollabfall usw.) im Innern zum Kopf der Person übertragen werden.

4. Apparat nach einem der vorhergehenden Ansprüche, gekennzeichnet durch eine Pumpe (74) und ein Ventil (75), das die Größe des Drucks im Innern und dadurch den auf den Kopf des Patienten ausgeübten Druck allein oder in Kombination mit der Erzeugung von Vibrationen durch den Oszillator steuert.

5. Apparat nach einem der vorhergehenden Ansprüche, dadurch gekennzeichnet, daß die Druckmittel auf einer zu dem Boden (M) parallelen Schiene (61) mittels einer Riemenscheibe (62) befestigt sind, so daß die Druckmittel entlang der Schiene (61) unterhalb von dieser bewegt werden können und die Person entlang der Schiene (61) unterhalb von dieser gehen kann, während das Kopfkontaktglied eine Wirbelzusammendrückkraft ausübt.

6. Apparat nach einem der vorhergehenden Ansprüche, dadurch gekennzeichnet, daß die Druckmittel geeignet sind, eine Kraft zwischen 3 % und 75 % des Körpergewichts der Person auszuüben.

7. Apparat nach einem der vorhergehenden Ansprüche, gekennzeichnet durch Mittel (120) zum Ausüben eines Druckes auf den Körper des Patienten, während die Wirbelsäule des Patienten in einer Weise zusammengedrückt wird, daß die Konfiguration der Wirbelsäule des Patienten korrigiert wird, während die Wirbelsäule zusammengedrückt wird.

8. Apparat nach Anspruch 7, dadurch gekennzeichnet, daß die Mittel (120,130) zum Ausüben eines Druckes auf die Wirbelsäule des Patienten unabhängige Drücke ausüben, um die Konfiguration der Schwerpunktposition des Patienten auf der Grundlage der Schwerpunktpositions-Daten des Patienten zu korrigieren.

9. Apparat nach Anspruch 7, dadurch gekennzeichnet, daß die Körperdruck-Ausübungsmittel linke und rechte Schulterdruckmittel (120,123a,123b,124) aufweisen, die unterschiedliche Drücke auf die linke bzw. rechte Schulter der Person ausüben können, wobei jede der linken und rechten Schulterdruckmittel einen Oszillator (127) enthalten.

10. Apparat nach einem der Ansprüche 7 und 9, dadurch gekennzeichnet, daß der kombinierte Druck, der sowohl von den Druckmitteln als auch den Körperdruck-Ausübungsmitteln ausgeübt wird, im Bereich von 50 % - 90 % des Körpergewichts der Person ist.

11. Apparat nach einem der Ansprüche 7, 9 und 10, dadurch gekennzeichnet, daß die Körperdruck-Ausübungsmittel ein Paar von bogenförmigen Führungsspuren (147a,147a) aufweisen, auf welchen linke und rechte Fußdruckglieder (144,144) jeweils bewegbar befestigt sind, wobei jedes der linken und rechten Fußdruckglieder individuell einstellbar ist, damit unterschiedliche Kräfte auf die Füße der Person ausgeübt werden können.

## Revendications

1. Appareil pour corriger le centre de gravité d'une personne humaine qui a au moins un corps (1), une tête (2) et une colonne vertébrale (6), comportant:
un élément (8, 13, 32, 63, 70, 115) de contact avec la tête qui est placé en contact avec la tête (2) de la personne, et
des moyens de compression (9, 14, 15, S, 33, 34, 36, 38, 64, 73, 74, 111a, 111b, 112a, 112b, 113, 114, 114a, 115) pour contraindre ledit élément de contact avec la tête contre la tête (2) de façon à comprimer au moins une portion de la colonne vertébrale (6),
caractérisé par le fait que ledit élément de contact avec la tête (8, 13, 32, 63, 70, 115) comporte un intérieur (8c, 13a, 32b, 70b, 114a) qui est rempli d'une substance fluide pour appliquer à la tête (2) de la personne une pression de surface essentiellement uniforme, l'intérieur (8c, 13a, 32b, 70b, 114a) et la substance fluide (8b, 14, 37, 73, 117) faisant partie de moyens qui appliquent à la tête (2) de la personne une pression de surface essentiellement uniforme.

2. Appareil comme exposé dans la réalisation 1, caractérisé par le fait que lesdits moyens de compression comportent en outre un contrepoids (9d) qui est relié par un câble (9b) audit élément de contact avec la tête, le contrepoids étant variable pour réduire sélectivement la valeur de la pression appliquée, par l'intermédiaire dudit élément du contact avec la tête (8, 13, 32, 63, 70, 115) par le poids de la substance fluide se trouvant dans ledit intérieur.

3. Appareil comme exposé dans l'une quelconque des revendications précédentes, caractérisé par le fait que ledit élément de contact avec la tête (8, 13, 32, 41, 70, 115) comporte en outre un oscillateur (W) qui est disposé pour produire des vibrations qui sont transmises à la tête de la personne par l'intermédiaire de la substance fluide (air, eau, mercure, sable, limaille de fer, gel de gélatine, déchets de coton etc.,) placés dans ledit intérieur.

4. Appareil comme exposé dans l'une quelconque des revendications précédentes, caractérisé par une pompe (74) et une vanne (75) qui commandent la valeur de la pression dans ledit intérieur et donc la pression appliquée à la tête du patient, seules ou en combinaison avec la génération de vibrations par ledit oscillateur.

5. Appareil comme exposé dans l'une quelconque des revendications précédentes, caractérisé par le fait que lesdits moyens de compression sont montés sur un rail (61) parallèle au plancher (M) par l'intermédiaire d'une poulie (62) de façon que les moyens de compression puissent se déplacer le long et en-dessous du rail (61) et que la personne puisse marcher le long et au-dessous dudit rail (61) pendant que ledit élément de contact avec la tête applique une force de compression sur la colonne vertébrale.

6. Appareil comme exposé dans l'une quelconque des revendications précédentes, caractérisé par le fait que lesdits moyens de compression sont conçus pour appliquer une force valant entre 3% et 75% du poids du corps de la personne.

7. Appareil comme exposé dans l'une quelconque des revendications précédentes, caractérisé par des moyens (120) pour appliquer une pression au corps du patient pendant que la colonne vertébrale du patient est comprimée d'une façon qui corrige la configuration de la colonne vertébrale du patient pendant que ladite colonne vertébrale est comprimée.

8. Appareil comme exposé dans la revendication 7, caractérisé par le fait que lesdits moyens (120, 130) prévus pour appliquer une pression à la colonne vertébrale du patient appliquent des compressions indépendantes pour corriger la configuration de la position du centre de gravité du patient sur la base des données relatives à la position du centre de gravité fournies par le patient.

9. Appareil exposé dans la revendication 7, caractérisé par le fait que lesdits moyens d'application d'une pression sur le corps comportent des moyens (120, 123a, 123b, 124) de compression des épaules de gauche et de droite qui peuvent appliquer différentes compressions aux épaules de gauche et de droite de la personne, respectivement, un oscillateur (127) étant inclus dans chacun desdits moyens de compression des épaules de gauche et de droite.

10. Appareil comme exposé dans l'une quelconque des revendications 7 et 9, caractérisé par le fait que la compression combinée appliquée à la fois par lesdits moyens de compression et lesdits moyens d'application de la pression vaut de 50% à 90% du poids du corps de la personne.

11. Appareil comme exposé dans l'une quelconque des revendications 7, 9 et 10, caractérisé par le fait que lesdits moyens d'application d'une pression au corps comportent une paire de pistes de guidage en arc (147a, 147a) sur lesquelles sont respectivement montés, avec possibilité de déplacement, des éléments (144, 144) de compression des pieds de gauche et de droite, chacun des éléments de compression des pieds de gauche et de droite étant individuellement réglable de façon à permettre que des forces différentes s'appliquent sur les pieds de la personne.
